(19) Europäisches Patentamt
European Patent Office
Office européen des brevets

(11) EP 0 959 067 B1

(12) # EUROPÄISCHE PATENTSCHRIFT

(45) Veröffentlichungstag und Bekanntmachung des Hinweises auf die Patenterteilung:
**11.09.2002 Patentblatt 2002/37**

(21) Anmeldenummer: **99108843.6**

(22) Anmeldetag: **04.05.1999**

(51) Int Cl.[7]: **C07C 241/02**, C07C 243/22

(54) **Verfahren zur Herstellung von Arylhydrazinen**

Process for the preparation of aryl hydrazines

Procédé pour la préparation des arylhydrazines

(84) Benannte Vertragsstaaten:
**CH DE FR GB LI**

(30) Priorität: **19.05.1998 DE 19822316**

(43) Veröffentlichungstag der Anmeldung:
**24.11.1999 Patentblatt 1999/47**

(73) Patentinhaber: **Clariant GmbH**
**65929 Frankfurt am Main (DE)**

(72) Erfinder:
- **Schach, Thomas Dr.**
  **Mumbai 400034 (IN)**
- **Volk, Heinrich Dr.**
  **61118 Bad Vilbel (DE)**
- **Koch, Manfred Dr.**
  **65817 Eppstein-Niederjosbach (DE)**

(56) Entgegenhaltungen:
**WO-A-97/24316**
**DE-A- 2 244 237**
**DE-A- 19 501 948**

EP 0 959 067 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft ein vorteilhaftes Verfahren zur Herstellung von Arylhydrazinen durch Hydrolyse entsprechender Arylhydrazindisulfonate.

Arylhydrazine, insbesondere Phenylhydrazine spielen eine bedeutende Rolle als Zwischenprodukte bei der Herstellung von Pflanzenschutzmitteln (Triazolone), Pharmazeutika aus der Reihe der Pyrazolone wie Antipyrin oder Pyramidon (Beyer/Walter, Lehrbuch der organischen Chemie Seite 588, 21. Auflage (1988), S. Hirzel Verlag Stuttgart) und Pyrazolonfarbstoffe.

Der bislang wichtigste Weg zur Herstellung von Arylhydrazinen, insbesondere Phenylhydrazinen geht von den entsprechenden Arylaminen aus. Im folgenden sei diese Art der Synthese beispielhaft bei der Herstellung von Phenylhydrazin ausgehend von Anilin belegt. Der Syntheseweg ist durch die nachfolgenden Gleichungen (1) bis (4) in verkürzter Form schematisch zusammengefaßt:

(1) $C_6H_5NH_2 + HCl + NO^+ \rightarrow C_6H_5N_2^+Cl + H^+ + H_2O$

(2) 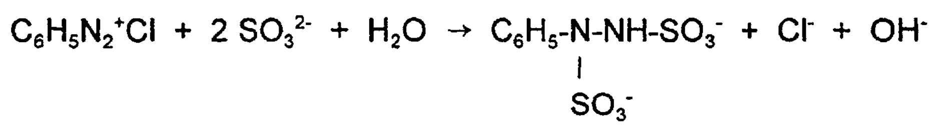

(3) 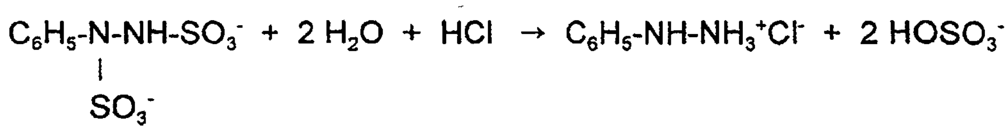

(4) $C_6H_5\text{-}NH\text{-}NH_3^+Cl + NaOH \rightarrow C_6H_5\text{-}NH\text{-}NH_2 + NaCl + H_2O$

Wie aus Gleichung (1) hervorgeht, wird Anilin in das entsprechende Diazoniumsalz umgewandelt, das gemäß Gleichung (2) mit Alkalisulfit zu dem entsprechenden Phenylhydrazindisulfonat reduziert wird. Das Phenylhydrazindisulfonat wird anschließend gemäß Gleichung (3) mit einer Mineralsäure hydrolysiert, wobei sich jedoch nicht das Phenylhydrazin, sondern das entsprechende Phenylhydrazinsalz bildet, aus dem sich, wie in Gleichung (4) beschrieben, das Phenylhydrazin durch eine Base, beispielsweise NaOH, freisetzen läßt. Diese Art der Herstellung von Phenylhydrazin ist in Houben-Weyl, Methoden der organischen Chemie, Band 10/2, S. 180 bis 191, insbesondere Seite 181 (1967), vierte Auflage, Georg Thieme Verlag, Stuttgart beschrieben.

Alle in den Gleichungen (1) bis (4) beschriebenen Reaktionsschritte werden in Wasser als Lösungsmittel durchgeführt. Insbesondere die durch Gleichung (3) beschriebene Hydrolyse, die mittels wäßriger Mineralsäure durchgeführt wird, erfolgt in Anwesenheit von Wasser.

Da es sich bei den gemäß den Gleichungen (1) bis (3) anfallenden Zwischenprodukten generell um Salze handelt, nämlich um ein Diazoniumsalz in Gleichung (1), um ein Hydrazindisulfonat in Gleichung (2) und um ein Hydrazinsalz in Gleichung (3), die im allgemeinen als gut wasserlöslich anzusehen sind, scheint der Einsatz eines zusätzlichen Lösungsmittels weder nützlich noch sinnvoll zu sein.

Im allgemeinen erfordert die in Gleichung (3) beschriebene Hydrolyse einen entsprechenden Überschuß an Mineralsäure, der in einer Reihe von Fällen sehr hoch sein kann. Ein hoher Überschuß an Mineralsäure führt jedoch ebenfalls zu einer hohen Belastung des bei der Hydrolyse anfallenden Abwassers und zu Korrosionsproblemen in den für die Hydrolyse verwendeten Reaktorbehältern. Ein hoher Anteil von Mineralsäure im Abwasser führt zudem zu einer deutlichen Steigerung der Kosten für die Abwasserbeseitigung und zu einem erhöhten technischen Aufwand für die Regeneration der Mineralsäure.

Es besteht daher ein Bedarf an einem Verfahren, das die vorstehend genannten Nachteile vermeidet und sich darüber hinaus, ohne großen apparativen Aufwand zu erfordern, auch in technischem Maßstab realisieren läßt.

Diese Aufgabe wird überraschenderweise gelöst durch ein Verfahren zur Herstellung von Arylhydrazinen der Formel $R^1R^2R^3Ar\text{-}NH\text{-}NH_2$ (I), indem man ein Arylhydrazindisulfonat der Formel

$$R^1R^2R^3Ar\text{-}N\text{-}NH\text{-}SO_3M^1 \text{ (II)},$$
|
$$SO_3M^2$$

worin $R^1$, $R^2$, $R^3$ gleich oder verschieden sind und H, $C_1$-$C_4$-Alkyl, $C_1$-$C_4$-Alkoxy, Halogen oder OH bedeuten, Ar Phenyl oder Naphthyl ist, $M^1$ und $M^2$ gleich oder verschieden sind und für H, $NH_4$, ein Alkalimetall oder ½ Erdalkalimetall stehen und $R^1$, $R^2$, $R^3$ und Ar in Formel (I) und (II) dieselbe Bedeutung haben, mit Wasser und einer anorganischen Säure bei 0 bis 100°C in Gegenwart eines inerten organischen Lösungsmittels zu dem entsprechenden Arylhydrazinsalz umsetzt und das Arylhydrazinsalz mit einer Base behandelt.

Die Anwesenheit des Lösungsmittels bei der Hydrolyse des Arylhydrazindisulfonats der Formel (II) führt in unerwarteter Weise zu Vorteilen bei der Hydrolyse. So kann in einer Reihe von Fällen, insbesondere bei instabilen Arylhydrazinsalzen, die einzusetzende Menge Mineralsäure reduziert werden. Ferner wird auch die Ausbeute und die Reinheit des Arylhydrazins verbessert.

Man kann in das erfindungsgemäße Verfahren mit gutem Erfolg ein Arylhydrazindisulfonat der Formel (II), worin Ar für Phenyl steht, einsetzen.

Es ist möglich, ein Arylhydrazindisulfonat der Formel (II), worin $R^1$, $R^2$, $R^3$ gleich oder verschieden und für H, $C_1$-$C_4$-Alkyl, Halogen oder OH, insbesondere für H, Halogen oder OH stehen, einsetzen. Als Halogen kommen insbesondere F, Cl oder Br, bevorzugt F oder Cl in Betracht.

In das erfindungsgemäße Verfahren kann man insbesondere ein Arylhydrazindisulfonat, worin ein oder zwei Reste $R^1$, $R^2$ und $R^3$ für OH oder Halogen stehen, insbesondere einer der Reste $R^1$, $R^2$ und $R^3$ für OH oder Halogen steht, einsetzen.

Gut geeignet als Arylhydrazindisulfonat der Formel (II) sind solche, in denen $M^1$ und $M^2$ gleich oder verschieden sind und für H, $NH_4$ oder ein Alkalimetall, insbesondere H, $NH_4$, Na oder K, bevorzugt Na oder K, besonders bevorzugt Na stehen.

Eine besondere Ausführungsform der vorliegenden Erfindung betrifft ein Verfahren zur Herstellung von 2,4-Dichlor-5-hydroxyphenylhydrazin, indem man 2,4-Dichlor-5-hydroxyphenylhydrazindisulfonat mit Wasser und einer Mineralsäure bei 0 bis 100, insbesondere 30 bis 70°C in Gegenwart eines inerten organischen Lösungsmittels zu dem entsprechenden 2,4-Dichlor-5-hydroxyphenylhydrazinsalz umsetzt, das 2,4-Dichlor-5-hydroxyphenylhydrazinsalz abtrennt und mit einer Base umsetzt.

Hierbei entspricht das 2,4-Dichlor-5-hydroxyphenylhydrazin dem Arylhydrazin der Formel (I) und das 2,4-Dichlor-5-hydroxyphenylhydrazindisulfonat dem Arylhydrazindisulfonat der Formel (II).

Man setzt als Arylhydrazindisulfonat der Formel (II) insbesondere 2,4-Dichlor-5-hydroxyphenylhydrazindisulfonat, worin $M^1$ und $M^2$ gleich oder verschieden sind und für H, $NH_4$ oder ein Alkalimetall, insbesondere H, $NH_4$, Na oder K, bevorzugt Na oder K, besonders bevorzugt Na stehen, ein.

Das 2,4-Dichlor-5-hydroxyphenylhydrazinsalz kann man als Feststoff oder vorzugsweise als organische Phase, die das 2,4-Dichlor-5-hydroxyphenylhydrazinsalz in dem organischen Lösungsmittel gelöst enthält, abtrennen.

Man setzt in dem erfindungsgemäßen Verfahren üblicherweise 2 bis 2000 mol Wasser je mol Arylhydrazindisulfonat der Formel (II) ein. In einer Vielzahl von Fällen hat es sich bewährt, 20 bis 1200, insbesondere 50 bis 1100, bevorzugt 100 bis 1000 mol Wasser je mol Arylhydrazindisulfonat zu verwenden.

Als anorganische Säure eignen sich Mineralsäuren. Man kann als anorganische Säure HCl, $H_2SO_4$, $H_3PO_4$ in wasserfreiem Zustand oder in wäßriger Lösung einsetzen. Der Gebrauch einer wäßrigen Lösung der anorganischen Säure ist besonders einfach.

Gut geeignet als anorganische Säure ist gasförmiges HCl oder eine wäßrige HCl-Lösung. Das Verfahren gestaltet sich besonders einfach bei Verwendung einer wäßrigen HCl-Lösung, insbesondere einer 5 bis 33%igen wäßrigen HCl-Lösung.

Man setzt 1 bis 30, insbesondere 2 bis 20, bevorzugt 4 bis 10 mol anorganische Säure je mol Arylhydrazindisulfonat ein.

Unter dem inerten organischen Lösungsmittel wird ein organisches Lösungsmittel verstanden, das sich unter den Reaktionsbedingungen inert verhält.

Als inertes organisches Lösungsmittel kann man einen aliphatischen Kohlenwasserstoff mit 5 bis 25 Kohlenstoffatomen, einen aromatischen Kohlenwasserstoff mit 6 bis 12 Kohlenstoffatomen, einen aliphatischen Alkohol mit 1 bis 12 Kohlenstoffatomen, ein Polyalkylenglykol mit 2 bis 6 Kohlenstoffatomen je Alkylen, einen Dialkylether mit 2 bis 20 Kohlenstoffatomen je Alkyl, ein Polyalkylenglykoldialkylether mit 1 bis 6 Kohlenstoffatomen je Alkylen und 1 bis 4 Kohlenstoffatomen je Alkyl, einen $C_1$-$C_4$-Alkylester einer aliphatischen $C_1$-$C_6$-Carbonsäure, ein aliphatisches $C_1$-$C_6$-Carbonsäuredi-($C_1$-$C_4$)-alkylamid, ein Nitril, ein Dialkylsulfoxid mit 1 bis 4 Kohlenstoffatomen je Alkyl, ein Dialkylsulfon mit 1 bis 4 Kohlenstoffatomen je Alkyl, ein Imidazolinon, ein Pyrrolidon oder ein Gemisch derselben einsetzen.

Gut geeignet als inertes organisches Lösungsmittel sind Methanol, Ethanol, n-Propanol, i-Propanol, n-Butanol, i-Butanol, tert.-Butanol, n-Pentanol, i-Pentanol, n-Hexanol, i-Hexanol, n-Octanol, i-Octanol, n-Decanol, i-Decanol, n-Dodecanol, Ethylenglykol, Glycerin, Diethylenglykol, Tetraethylenglykol, Diethylenglykoldimethylether, Tetraethylenglykoldimethylether, Dimethylformamid, Diethylformamid, Dimethylacetamid oder ein Gemisch derselben, insbesondere Methanol, Ethanol, n-Propanol, i-Propanol, n-Butanol, i-Butanol, tert.-Butanol, n-Pentanol, i-Pentanol, n-Hexanol, i-Hexanol, n-Octanol, i-Octanol, n-Decanol, i-Decanol oder ein Gemisch derselben.

Besonders geeignet sind als inertes organisches Lösungsmittel n-Butanol, i-Butanol, n-Pentanol, i-Pentanol, n-Hexanol, i-Hexanol, n-Octanol, i-Octanol, n-Decanol, i-Decanol oder ein Gemisch derselben. In einigen Fällen hat sich beispielsweise n-Butanol, i-Butanol, n-Pentanol, i-Pentanol, n-Hexanol, i-Hexanol, n-Octanol, i-Octanol oder ein Gemisch derselben, insbesondere n-Butanol, i-Butanol, n-Pentanol, i-Pentanol, n-Hexanol, i-Hexanol oder ein Gemisch derselben, bevorzugt n-Butanol, i-Butanol, n-Pentanol, i-Pentanol oder ein Gemisch derselben als inertes organisches Lösungsmittel bewährt.

Man setzt üblicherweise 0,01 bis 100 mol inertes organisches Lösemittel je mol eingesetztes Arylhydrazindisulfonat ein. In einer Vielzahl von Fällen hat es sich bewährt 1 bis 50, insbesondere 10 bis 30 mol inertes organisches Lösungsmittel je mol eingesetztes Arylhydrazindisulfonat einzusetzen.

Das inerte organische Lösungsmittel kann zwei verschiedene Wirkungen ausüben. Zum einen kann es zu einer Beschleunigung der Umsetzung (Hydrolyse) führen und zum anderen kann es das gebildete Arylhydrazinsalz bereits während der Bildung aus der wäßrigen Phase extrahieren.

Ist die Beschleunigung der Umsetzung (Hydrolyse) gewünscht, so lassen sich mit gutem Erfolg Ethylenglykol, Glycerin, Diethylenglykol, Tetraethylenglykol, Diethylenglykoldimethylether, Tetraethylenglykoldimethylether, Dimethylformamid, Dimethylacetamid oder ein Gemisch derselben, insbesondere Ethylenglykol, Diethylenglykol, Diethylenglykoldimethylether einsetzen.

Diese Beschleunigung der Umsetzung läßt sich bereits mit vergleichsweise geringen Mengen Lösungsmittel erreichen. Üblicherweise setzt man hierbei 0,1 bis 5, insbesondere 0,5 bis 3 mol inertes organisches Lösungsmittel je mol eingesetztes Arylhydrazindisulfonat ein.

Beabsichtigt man, das Arylhydrazinsalz bereits während seiner Bildung in der Hydrolyse aus der wäßrigen Phase zu extrahieren, so lassen sich mit gutem Erfolg aliphatische Alkohole mit 4 bis 10 Kohlenstoffatomen, beispielsweise n-Butanol, i-Butanol, n-Hexanol, n-Decanol verwenden.

Zur Durchführung dieser Extraktion setzt man das inerte organische Lösungsmittel in entsprechenden Mengen zu. Üblicherweise verwendet man 1 bis 50, insbesondere 5 bis 30, bevorzugt 10 bis 20 mol inertes organisches Lösungsmittel je mol eingesetztes Arylhydrazindisulfonat.

Es hat sich als günstig erwiesen, Wasser, die anorganische Säure und das inerte organische Lösungsmittel vorzulegen und das Arylhydrazindisulfonat der Formel (II) zuzugeben.

Bei Durchführen der Umsetzung empfiehlt es sich, die Geschwindigkeit der Zugabe des Arylhydrazindisulfonats zu Wasser, anorganischer Säure und inertem organischen Lösungsmittel angemessen zu wählen. Eine zu hohe Geschwindigkeit der Zugabe des Arylhydrazindisulfonats kann zu einer Verringerung der Ausbeute an Arylhydrazin führen. Im Labormaßstab hat sich eine Zugabe des Arylhydrazindisulfonats innerhalb eines Zeitraumes von 5 Minuten bis 5 Stunden, insbesondere 10 Minuten bis 3 Stunden als ausreichend erwiesen.

Man erwärmt das Wasser, die anorganische Säure und das inerte organische Lösungsmittel unter Rühren auf die gewünschte Reaktionstemperatur und gibt dann das Arylhydrazindisulfonat zu. Besonders günstig ist es, das Arylhydrazindisulfonat in Form einer wäßrigen Lösung zu verwenden.
In einer Vielzahl von Fällen hat sich eine Reaktionstemperatur von 10 bis 90, insbesondere 40 bis 80°C als ausreichend erwiesen.

Während der Zugabe des Arylhydrazindisulfonats und der sich daran anschließenden Nachreaktion ist für eine gute Durchmischung des sich bildenden Reaktionsgemisches zu sorgen.

Im Anschluß an die Hydrolyse des Arylhydrazindisulfonats liegt das entsprechende Arylhydrazinsalz vor, das im Reaktionsgemisch verbleibt oder aus dem Reaktionsgemisch abgetrennt wird.

Man kann das Arylhydrazinsalz als organische Phase, die das Arylhydrazinsalz in dem inerten organischen Lösungsmittel gelöst enthält, abtrennen. Dies erfolgt durch eine einfache Phasentrennung und stellt eine besonders günstige Variante des erfindungsgemäßen Verfahrens dar.

Es ist auch möglich, das Arylhydrazinsalz als Feststoff, beispielsweise durch Filtration, Sedimentation oder Zentrifugieren, abzutrennen.

Aus dem abgetrennten Arylhydrazinsalz oder aus dem das Arylhydrazinsalz enthaltenden Reaktionsgemisch wird anschließend durch Zugabe der Base das Arylhydrazin freigesetzt. Als Base eignen sich Alkalioxide, Alkalihydroxide, Alkalicarbonate, Erdalkalioxide, Erdalkalihydroxide in festem Zustand oder in Form wäßriger Lösungen.

Besonders einfach ist es, das Arylhydrazinsalz mit einer wäßrigen Alkalihydroxidlösung, insbesondere einer wäßrigen NaOH und/oder KOH Lösung, bevorzugt einer wäßrigen NaOH Lösung zu behandeln.

Die Zugabe der Base erfolgt üblicherweise bei 0 bis 50, insbesondere 5 bis 40°C.

Das erfindungsgemäße Verfahren läßt sich kontinuierlich oder diskontinuierlich, insbesondere diskontinuierlich durchführen.

Die nachfolgenden Beispiele beschreiben die vorliegende Erfindung näher, ohne sie zu beschränken.

## Experimenteller Teil

### Beispiel 1

Herstellung von 2,4-Dichlor-5-hydroxyphenylhydrazin unter Zusatz von n-Butanol bei 50°C

In einem 4 l Vierhalskolben mit Tropftrichter, Rührer und Kühler werden 417,8 g wäßrige HCl (30 %ig), entsprechend 3,4 mol HCl, und 422,4 g n-Butanol (wassergesättigt, ca. 80 %ig), entsprechend 4,6 mol n-Butanol, vorgelegt. Die Lösung wird auf 50°C erwärmt und innerhalb von 2 Stunden mit insgesamt 1394,6 g einer wäßrigen 2,4-Dichlor-5-hydroxyphenylhydrazindisulfonat-Lösung (10,3 %ig), entsprechend 0,36 mol Dinatrium-2,4-dichlor-5-hydroxyphenylhydrazindisulfonat, versetzt. Die Reaktionslösung wird noch weitere 60 Minuten bei 50°C gehalten. Das Reaktionssystem bildet im Laufe der Umsetzung zwei Phasen. Eine obere organische Phase und eine untere wäßrige Phase. Beide Phasen werden in einem 4 l Scheidetrichter getrennt. Die wäßrige Phase wird anschließend dreimal mit je 50 ml Butanol extrahiert. Die organischen Phasen werden vereinigt und mit wäßriger Natronlauge (33 %ig) auf pH 2 - 2,5 eingestellt und die sich dabei bildende wäßrige Phase wird ebenfalls abgetrennt. Nach der Zugabe von 142 g Wasser wird mit wäßriger Natronlauge (33 %ig) der pH auf 6,3 - 6,9 eingestellt und das kristalline 2,4-Dichlor-5-hydroxyphenylhydrazin als orangefarbener Feststoff bei 15 - 20°C über eine 500 ml Nutsche abgesaugt, mit 218 g Butanol gewaschen und getrocknet.

Ausbeute: 68,9 g (0,34 mol) 2,4-Dichlor-5-hydroxyphenylhydrazin (96 %ig), das entspricht einer theoretischen Ausbeute von 96,0 % bezogen auf eingesetztes 2,4-Dichlor-5-hydroxyphenylhydrazindisulfonat.

Die Ausbeute ist signifikant höher als die im Vergleichsbeispiel 1 erzielte Ausbeute.

### Beispiel 1A

Herstellung von 2,4-Dichlor-5-hydroxyphenylhydrazin unter Zusatz von Ethylenglykol bei 50°C

In einem 4 l Vierhalskolben mit Tropftrichter, Rührer und Kühler werden 417 g (3,4 mol) HCl 30 %ig und 58 g (0,93 mol) Ethylenglykol vorgelegt. Die Lösung wird auf 50°C erwärmt und innerhalb von 2 Stunden mit insgesamt 1394,6 g (0,36 mol) 2,4-Dichlor-5-hydroxyphenylhydrazindisulfonat 10,3 %ig versetzt. Die Reaktionslösung wird für weitere 60 Minuten bei 50°C gehalten und anschließend auf Raumtemperatur gekühlt. Sofort nach Beginn der Dosierung kristallisiert ein heller Feststoff aus der Reaktionslösung. Es bildet sich ein sehr voluminöser Kristallbrei, der nach dem Abkühlen auf 15 - 20°C über eine 1 l Saugnutsche abfiltriert wird.

Ausbeute: 121,0 g (0,19 mol) 2,4-Dichlor-5-hydroxyphenylhydrazinhydrochlorid (35,0 %ig), das entspricht einer theoretischen Ausbeute von 51,8 % bezogen auf eingesetztes 2,4-Dichlor-5-hydroxyphenylhydrazindisulfonat.

Das erhaltene 2,4-Dichlor-5-hydroxyphenylhydrazinhydrochlorid wird anschließend in Wasser aufgenommen und mit NaOH 33 %ig auf pH 6,3 - 6,8 eingestellt. Der sich bildende Niederschlag wird anschließend in einer 250 ml Saugnutsche abgetrennt, mit Wasser nachgewaschen und getrocknet.

Ausbeute: 38,8 g (0,18 mol) 2,4-Dichlor-5-hydroxyphenylhydrazin (90 %ig) das entspricht einer theoretischen Ausbeute von 50,3 % bezogen auf eingesetztes 2,4-Dichlor-5-hydroxyphenylhydrazindisulfonat und 96,0 % bezogen auf zwischenisoliertes 2,4-Dichlor-5-hydroxyphenylhydrazinhydrochlorid.

Die Ausbeute liegt deutlich über der in Vergleichsbeispiel 1 erzielten Ausbeute.

### Vergleichsbeispiel 1

Herstellung von 2,4-Dichlor-5-hydroxyphenylhydrazin ohne Lösungsmittelzusatz bei 50°C

In einem 4 l Vierhalskolben mit Tropftrichter, Rührer und Kühler werden 417 g wäßrige HCl (30 %ig), entsprechend 3,4 mol, vorgelegt. Die HCl (30 %ig) wird auf 50°C erwärmt und innerhalb von 2 Stunden mit insgesamt 1394,6 g einer wäßrigen 2,4-Dichlor-5-hydroxyphenylhydrazindisulfonat-Lösung (10,3 %ig), entsprechend 0,36 mol, versetzt. Die Reaktionslösung wird noch weitere 60 Minuten bei 50°C gehalten und anschließend auf Raumtemperatur gekühlt. Sofort nach Beginn der Dosierung kristallisiert ein heller Feststoff aus der Reaktionslösung. Es bildet sich ein sehr voluminöser Kristallbrei, der nach dem Abkühlen auf 15 - 20°C über eine 1 l Saugnutsche abfiltriert wird.

Ausbeute: 109,1 g (0,154 mol) 2,4-Dichlor-5-hydroxyphenylhydrazinhydrochlorid (32,0 %ig), das entspricht einer theoretischen Ausbeute von 42,7 % bezogen auf eingesetztes 2,4-Dichlor-5-hydroxyphenylhydrazindisulfonat.

Das erhaltene 2,4-Dichlor-5-hydroxyphenylhydrazinhydrochlorid wird anschließend in Wasser aufgenommen und mit wäßriger NaOH (33 %ig) auf pH 6,3 - 6,8 eingestellt. Der sich bildende Niederschlag wird anschließend in einer 100 ml Saugnutsche abgetrennt, mit Wasser nachgewaschen und getrocknet.
Ausbeute: 31,3 g (0,147 mol) 2,4-Dichlor-5-hydroxyphenylhydrazin (91 %ig), das entspricht einer theoretischen Ausbeute von 41,0 % bezogen auf eingesetztes 2,4-Dichlor-5-hydroxyphenylhydrazindisulfonat.

Beispiel 2

Herstellung von 2,4-Dichlor-5-hydroxyphenylhydrazin unter Zusatz von n-Butanol bei 60°C

In einem 4 l Vierhalskolben mit Tropftrichter, Rührer und Kühler werden 417,8 g wäßrige HCl (30 %ig), entsprechend 3,4 mol, und 422,4 g n-Butanol (wassergesättigt, ca. 80 %ig), entsprechend 4,6 mol, vorgelegt. Die Lösung wird auf 60°C erwärmt und innerhalb von 15 Minuten mit insgesamt 1394,6 g einer wäßrigen 2,4-Dichlor-5-hydroxyphenylhydrazindisulfonat-Lösung (10,3 %ig), entsprechend 0,36 mol, versetzt. Die Reaktionslösung wird noch weitere 60 Minuten bei 60°C gehalten und anschließend rasch auf 40°C gekühlt. Das Reaktionssystem bildet im Laufe der Umsetzung zwei Phasen. Eine obere organische Phase und eine untere wäßrige Phase. Beide Phasen werden in einem 4 l Scheidetrichter getrennt. Die wäßrige Phase wird anschließend dreimal mit je 50 ml Butanol extrahiert. Die organischen Phasen werden vereinigt und mit wäßriger Natronlauge (33 %ig) auf pH 2 - 2,5 eingestellt und die sich dabei bildende wäßrige Phase wird ebenfalls abgetrennt. Nach der Zugabe von 142 g Wasser wird mit wäßriger Natronlauge (33 %ig) der pH auf 6,3 - 6,9 eingestellt und das kristalline 2,4-Dichlor-5-hydroxyphenylhydrazin als orangefarbener Feststoff bei 15 - 20°C über eine 500 ml Nutsche abgesaugt, mit 218 g Butanol gewaschen und getrocknet.
Ausbeute: 64,7 g (0,31 mol) 2,4-Dichlor-5-hydroxyphenylhydrazin (93 %ig), das entspricht einer theoretischen Ausbeute von 87 % bezogen auf eingesetztes 2,4-Dichlor-5-hydroxyphenylhydrazindisulfonat.
Die Ausbeute ist in Relation zu dem Ergebnis von Vergleichsbeispiel 2 (keine isolierbare Ausbeute) zu setzen.

Vergleichsbeispiel 2

Herstellung von 2,4-Dichlor-5-hydroxyphenylhydrazin ohne Lösungsmittelzusatz bei 60°C

In einem 4 l Vierhalskolben mit Tropftrichter, Rührer und Kühler werden 417,8 g wäßrige HCl (30 %ig), entsprechend 3,4 mol, vorgelegt. Die HCl (30 %ig) wird auf 60°C erwärmt und innerhalb von 15 Minuten mit insgesamt 1394,6 g einer wäßrigen 2,4-Dichlor-5-hydroxyphenylhydrazindisulfonat-Lösung (10,3 %ig), entsprechend 0,36 mol, versetzt. Die Reaktionslösung wird noch weitere 2 Stunden bei 60°C gehalten und anschließend auf Raumtemperatur gekühlt. Bereits beim Zudosieren färbt sich die Reaktionslösung tiefbraun und am Ende der Nachrührphase lassen sich geringe Mengen eines teerigen Rückstandes isolieren.
Ausbeute: Kein isolierbares 2,4-Dichlor-5-hydroxyphenylhydrazinhydrochlorid.

Beispiel 3

Herstellung von 2,4-Dichlor-5-hydroxyphenylhydrazin unter Zusatz von n-Butanol bei 70°C

In einem 4 l Vierhalskolben mit Tropftrichter, Rührer und Kühler werden 417,8 g wäßrige HCl (30 %ig), entsprechend 3,4 mol, und 422,4 g n-Butanol (wassergesättigt, ca. 80 %ig), entsprechend 4,6 mol, vorgelegt. Die Lösung wird auf 70°C erwärmt und innerhalb von 10 Minuten mit insgesamt 1394,6 g einer wäßrigen 2,4-Dichlor-5-hydroxyphenylhydrazindisulfonat-Lösung (10,3 %ig), entsprechend 0,36 mol, versetzt. Die Reaktionslösung wird noch weitere 45 Minuten bei 70°C gehalten. Das Reaktionssystem bildet im Laufe der Umsetzung zwei Phasen. Eine obere organische Phase und eine untere wäßrige Phase.Beide Phasen werden in einem 4 l Scheidetrichter getrennt. Die wäßrige Phase wird anschließend dreimal mit je 50 ml Butanol extrahiert. Die organischen Phasen werden vereinigt und mit wäßriger Natronlauge (33 %ig) auf pH 2 - 2,5 eingestellt und die sich dabei bildende wäßrige Phase wird ebenfalls abgetrennt. Nach der Zugabe von 142 g Wasser wird mit wäßriger Natronlauge (33 %ig) der pH auf 6,3 - 6,9 eingestellt und das kristalline 2,4-Dichlor-5-hydroxyphenylhydrazin als orangefarbener Feststoff bei 15 - 20°C über eine 500 ml Nutsche abgesaugt, mit 218 g Butanol gewaschen und getrocknet.
Ausbeute: 70,2 g (0,34 mol) 2,4-Dichlor-5-hydroxyphenylhydrazin (93,1 %ig), das entspricht einer theoretischen Ausbeute von 94,5 % bezogen auf eingesetztes 2,4-Dichlor-5-hydroxyphenylhydrazindisulfonat.
Die Ausbeute ist in Relation zu dem Ergebnis von Vergleichsbeispiel 3 (keine isolierbare Ausbeute) zu setzen.

Vergleichsbeispiel 3

In einem 4 l Vierhalskolben mit Tropftrichter, Rührer und Kühler werden 417,8 g wäßrige HCl (30 %ig) vorgelegt. Die HCl (30 %ig) wird auf 70°C erwärmt und innerhalb von 15 Minuten mit insgesamt 1394,6 g einer wäßrigen 2,4-Dichlor-5-hydroxyphenylhydrazindisulfonat-Lösung (10,3 %ig) versetzt. Die Reaktionslösung wird noch weitere 45 Minuten bei 70°C gehalten und anschließend auf Raumtemperatur gekühlt. Bereits beim Zudosieren färbt sich die Reaktionslösung tiefbraun und am Ende der Nachrührphase lassen sich nur noch polymere Zersetzungsprodukte isolieren.

Ausbeute: Kein isolierbares 2,4-Dichlor-5-hydroxyphenylhydrazinhydrochlorid.

Beispiel 4

Herstellung von 2-Fluorphenylhydrazin unter Zusatz von n-Butanol

In einem 2 l Vierhalskolben mit Tropftrichter, Rührer und Kühler werden 240 g HCl 30 %ig und 180 g n-Butanol (wassergesättigt, ca. 80 %ig) vorgelegt. Die Lösung wird auf 50°C erwärmt und innerhalb von 25 Minuten mit insgesamt 1291 g wäßriger 2-Fluorphenylhydrazindisulfonatlösung - hergestellt durch Diazotierung von 111 g (1 mol) 2-Fluoranilin (156 g Wasser, 303 g HCl 30 %ig, 170 g $NaNO_2$) und anschließender Sulfitreduktion (560 g $NaHSO_3$ + 100 g NaOH) - versetzt. Die Reaktionslösung wird noch weitere 60 Minuten bei 50°C gehalten. Das Reaktionssystem bildet im Laufe der Umsetzung zwei Phasen. Beide Phasen werden in einem 2 l Scheidetrichter voneinander getrennt. Die wäßrige Phase wird anschließend dreimal mit je 10 ml n-Butanol extrahiert. Die organischen Phasen werden vereinigt und mit Natronlauge (33 %ig) auf pH 8,5 eingestellt und die sich dabei bildende wäßrige Phase wird ebenfalls abgetrennt. Das Lösungsmittel wird anschließend soweit eingeengt, bis sich ein zäher Kristallbrei bildet.

Ausbeute: 98,2 g (0,74 mol) 2-Fluorphenylhydrazin (95 %ig), das entspricht einer theoretischen Ausbeute von 74,0 %, bezogen auf eingesetztes 2-Fluoranilin.

Beispiel 5

Herstellung von 4-Chlorphenylhydrazin unter Zusatz von n-Butanol

In einem 2 l Vierhalskolben mit Tropftrichter, Rührer und Kühler werden 95 g HCl 30 %ig und 57 g n-Butanol (wassergesättigt, ca. 80 %ig) vorgelegt. Die Lösung wird auf 50°C erwärmt und innerhalb von 2 Stunden mit insgesamt 720 g wäßriger 4-Chlorphenylhydrazindisulfonatlösung - hergestellt durch Diazotierung von 34,4 g (0,27 mol) 4-Chloranilin (100 g Wasser, 78 g HCl 30 %ig, 55 g $NaNO_2$) und anschließender Sulfitreduktion (186 g $NaHSO_3$ + 57 g NaOH) - versetzt. Die Reaktionsemulsion wird noch weitere 60 Minuten bei 50°C gehalten, mit 119 g Natronlauge 33 %ig auf pH 6,9 eingestellt und die sich dabei bildende wäßrige Phase abgetrennt. Die wäßrige Phase wird dreimal mit je 5 ml n-Butanol extrahiert und die vereinigten organischen Phasen werden auf 15°C gekühlt. Das sich bildende 4-Chlorphenylhydrazin wird als kristalliner Feststoff bei 15°C über eine 500 ml Nutsche abgesaugt, mit 50 ml n-Butanol gewaschen und getrocknet.

Ausbeute: 36,2 g (0,21 mol) 4-Chlorphenylhydrazin (82,5 %ig), das entspricht einer theoretischen Ausbeute von 78,0 %, bezogen auf eingesetztes 4-Chloranilin.

## Patentansprüche

1. Verfahren zur Herstellung von Arylhydrazinen der Formel $R^1R^2R^3Ar\text{-}NH\text{-}NH_2$ (I), indem man ein Arylhydrazindisulfonat der Formel

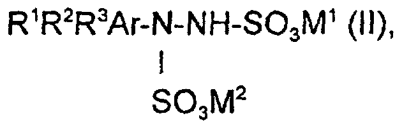

worin $R^1$, $R^2$, $R^3$ gleich oder verschieden sind und H, $C_1$-$C_4$-Alkyl, $C_1$-$C_4$-Alkoxy, Halogen oder OH bedeuten, Ar Phenyl oder Naphthyl ist, $M^1$ und $M^2$ gleich oder verschieden sind und für H, $NH_4$, ein Alkalimetall oder ½ Erdalkalimetall stehen und $R^1$, $R^2$, $R^3$ und Ar in Formel (I) und (II) dieselbe Bedeutung haben, mit Wasser und einer

anorganischen Säure bei 0 bis 100°C in Gegenwart eines inerten organischen Lösungsmittels zu dem entsprechenden Arylhydrazinsalz umsetzt und das Arylhydrazinsalz mit einer Base behandelt.

2. Verfahren nach Anspruch 1, **dadurch gekennzeichnet, daß** man ein Arylhydrazindisulfonat der Formel (II), worin Ar für Phenyl steht, einsetzt.

3. Verfahren nach Anspruch 1 oder 2, **dadurch gekennzeichnet, daß** man ein Arylhydrazindisulfonat der Formel (II), worin ein oder zwei Reste $R^1$, $R^2$ und $R^3$ für OH oder Halogen stehen, einsetzt.

4. Verfahren nach einem oder mehreren der Ansprüche 1 bis 3, **dadurch gekennzeichnet, daß** man als Arylhydrazindisulfonat der Formel (II) 2,4-Dichlor-5-hydroxyphenylhydrazindisulfonat, worin $M^1$ und $M^2$ gleich oder verschieden sind und für H, $NH_4$ oder ein Alkalimetall stehen, einsetzt.

5. Verfahren nach einem oder mehreren der Ansprüche 1 bis 4, **dadurch gekennzeichnet, daß** man 2 bis 2000 mol Wasser je mol Arylhydrazindisulfonat einsetzt.

6. Verfahren nach einem oder mehreren der Ansprüche 1 bis 5, **dadurch gekennzeichnet, daß** man als anorganische Säure HCl, $H_2SO_4$, $H_3PO_4$ in wasserfreiem Zustand oder in wäßriger Lösung einsetzt.

7. Verfahren nach einem oder mehreren der Ansprüche 1 bis 6, **dadurch gekennzeichnet, daß** man 1 bis 30 mol anorganische Säure je mol Arylhydrazindisulfonat einsetzt.

8. Verfahren nach einem oder mehreren der Ansprüche 1 bis 7, **dadurch gekennzeichnet, daß** man als inertes organisches Lösungsmittel einen aliphatischen Kohlenwasserstoff mit 5 bis 25 Kohlenstoffatomen, einen aromatischen Kohlenwasserstoff mit 6 bis 12 Kohlenstoffatomen, einen aliphatischen Alkohol mit 1 bis 12 Kohlenstoffatomen, ein Polyalkylenglykol mit 2 bis 6 Kohlenstoffatomen je Alkylen, einen Dialkylether mit 2 bis 20 Kohlenstoffatomen je Alkyl, ein Polyalkylenglykoldialkylether mit 1 bis 6 Kohlenstoffatomen je Alkylen und 1 bis 4 Kohlenstoffatomen je Alkyl, einen $C_1$-$C_4$-Alkylester einer aliphatischen $C_1$-$C_6$-Carbonsäure, ein aliphatisches $C_1$-$C_6$-Carbonsäuredi-($C_1$-$C_4$)-alkylamid, ein Nitril, ein Dialkylsulfoxid mit 1 bis 4 Kohlenstoffatomen je Alkyl, ein Dialkylsulfon mit 1 bis 4 Kohlenstoffatomen je Alkyl, ein Imidazolinon, ein Pyrrolidon oder ein Gemisch derselben einsetzt.

9. Verfahren nach einem oder mehreren der Ansprüche 1 bis 8, **dadurch gekennzeichnet, daß** man als inertes organisches Lösungsmittel Methanol, Ethanol, n-Propanol, i-Propanol, n-Butanol, i-Butanol, tert.-Butanol, n-Pentanol, i-Pentanol, n-Hexanol, i-Hexanol, n-Octanol, i-Octanol, n-Decanol, i-Decanol, n-Dodecanol, Ethylenglykol, Glycerin, Diethylenglykol, Tetraethylenglykol, Diethylenglykoldimethylether, Tetraethylenglykoldimethylether, Dimethylformamid, Diethylformamid, Dimethylacetamid oder ein Gemisch derselben einsetzt.

10. Verfahren nach einem oder mehreren der Ansprüche 1 bis 9, **dadurch gekennzeichnet, daß** man als inertes organisches Lösungsmittel Methanol, Ethanol, n-Propanol, i-Propanol, n-Butanol, i-Butanol, tert.-Butanol, n-Pentanol, i-Pentanol, n-Hexanol, i-Hexanol, n-Octanol, i-Octanol, n-Decanol, i-Decanol oder ein Gemisch derselben einsetzt.

11. Verfahren nach einem oder mehreren der Ansprüche 1 bis 10, **dadurch gekennzeichnet, daß** man das Arylhydrazinsalz als organische Phase, die das Arylhydrazinsalz in dem organischen Lösungsmittel gelöst enthält, abtrennt.

## Claims

1. A process for preparing arylhydrazines of the formula
$R^1R^2R^3Ar\text{-}NH\text{-}NH_2$ (I) by reacting an arylhydrazinedisulfonate of the formula

$$R^1R^2R^3Ar\text{-}\underset{\substack{|\\ SO_3M^2}}{N}\text{-}NH\text{-}SO_3M^1 \text{ (II),}$$

in which $R^1$, $R^2$, and $R^3$ are identical or different and are H, $C_1$-$C_4$-alkyl, $C_1$-$C_4$-alkoxy, halogen or OH, Ar is phenyl or naphthyl, $M^1$ and $M^2$ are identical or different and are H, $NH_4$, an alkali metal or % alkaline earth metal and $R^1$, $R^2$, $R^3$ and Ar in the formulae (I) and (II) have the same meaning, with water and an inorganic acid at from 0 to 100°C in the presence of an inert organic solvent to give the corresponding arylhydrazine salt and treating the arylhydrazine salt with a base.

**2.** The process as claimed in claim 1, wherein an arylhydrazinedisulfonate of the formula (II), in which Ar is phenyl, is employed.

**3.** The process as claimed in claim 1 or 2, wherein an arylhydrazinedisulfonate of the formula (II), in which one or two radicals $R^1$, $R^2$ and $R^3$ are OH or halogen, is employed.

**4.** The process as claimed in one or more of claims 1 to 3, wherein the arylhydrazinedisulfonate of the formula (II) employed is 2,4-dichloro-5-hydroxyphenylhydrazinedisulfonate in which $M^1$ and $M^2$ are identical or different and are H, $NH_4$ or an alkali metal.

**5.** The process as claimed in one or more of claims 1 to 4, wherein from 2 to 2000 mol of water are employed per mole of arylhydrazinedisulfonate.

**6.** The process as claimed in one or more of claims 1 to 5, wherein the inorganic acid employed is HCl, $H_2SO_4$, $H_3PO_4$, in an anhydrous state or in an aqueous solution.

**7.** The process as claimed in one or more of claims 1 to 6, wherein from 1 to 30 mol of inorganic acid are employed per mole of arylhydrazinedisulfonate.

**8.** The process as claimed in one or more of claims 1 to 7, wherein the inert organic solvent used is an aliphatic hydrocarbon having from 5 to 25 carbon atoms, an aromatic hydrocarbon having from 6 to 12 carbon atoms, an aliphatic alcohol having from 1 to 12 carbon atoms, a polyalkylene glycol having from 2 to 6 carbon atoms per alkylene, a dialkyl ether having from 2 to 20 carbon atoms per alkyl, a polyalkylene glycol dialkyl ether having from 1 to 6 carbon atoms per alkylene and from 1 to 4 carbon atoms per alkyl, a $C_1$-$C_4$-alkyl ester of an aliphatic $C_1$-$C_6$-carboxylic acid, an aliphaticdi-($C_1$-$C_4$-alkyl)-$C_1$-$C_6$-carboxamide, a nitrile, a dialkyl sulfoxide having from 1 to 4 carbon atoms per alkyl, a dialkylsulfone having from 1 to 4 carbon atoms per alkyl, an imidazolinone, a pyrrolidone or a mixture of these.

**9.** The process as claimed in one or more of claims 1 to 8, wherein the inert organic solvent used is methanol, ethanol, n-propanol, isopropanol, n-butanol, isobutanol, tert-butanol, n-pentanol, isopentanol, n-hexanol, isohexanol, n-octanol, isooctanol, n-decanol, isodecanol, n-dodecanol, ethylene glycol, glycerol, diethylene glycol, tetraethylene glycol, diethylene glycol dimethyl ether, tetraethylene glycol dimethyl ether, dimethylformamide, diethylformamide, dimethylacetamide or a mixture of these.

**10.** The process as claimed in one or more of claims 1 to 9, wherein the inert organic solvent used is methanol, ethanol, n-propanol, isopropanol, n-butanol, isobutanol, tert-butanol, n-pentanol, isopentanol, n-hexanol, isohexanol, n-octanol, isooctanol, n-decanol, isodecanol or a mixture of these.

**11.** The process as claimed in one or more of claims 1 to 10, wherein the arylhydrazine salt is separated off as organic phase containing the arylhydrazine salt dissolved in the organic solvent.

## Revendications

**1.** Procédé de préparation d'arylhydrazines de formule $R^1R^2R^3Ar$-NH-$NH_2$ (I), dans lequel on fait réagir un disulfonate d'arylhydrazine de formule

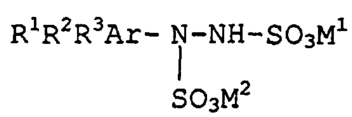
(II)

dans laquelle $R^1$, $R^2$ et $R^3$ sont identiques ou différents et représentent chacun H, un groupe alkyle en $C_1$-$C_4$, alcoxy en $C_1$-$C_4$, halogéno ou OH, Ar est le groupe phényle ou naphtyle, $M^1$ et $M^2$ sont identiques ou différents et représentent chacun H, $NH_4$, un métal alcalin ou un demi-métal alcalino-terreux, et $R^1$, $R^2$, $R^3$ et Ar, dans les formules (I) et (II) ont les mêmes significations, avec de l'eau et un acide inorganique à une température de 0 à 100°C, en présence d'un solvant organique inerte, pour obtenir le sel d'arylhydrazine correspondant, et on traite le sel d'arylhydrazine avec une base.

**2.** Procédé selon la revendication 1, **caractérisé en ce qu'**on utilise un disulfonate d'arylhydrazine de formule (II) dans laquelle Ar est le groupe phényle.

**3.** Procédé selon la revendication 1 ou 2, **caractérisé en ce qu'**on utilise un disulfonate d'arylhydrazine de formule (II) dans laquelle un ou deux radicaux $R^1$, $R^2$ et $R^3$ sont des groupes OH ou halogéno.

**4.** Procédé selon l'une ou plusieurs des revendications 1 à 3, **caractérisé en ce qu'**on utilise en tant que disulfonate d'arylhydrazine de formule (II) le disulfonate de 2,4-dichloro-5-hydroxyphénylhydrazine, où $M^1$ et $M^2$ sont identiques ou différents et représentent chacun H, $NH_4$ ou un métal alcalin.

**5.** Procédé selon l'une ou plusieurs des revendications 1 à 4, **caractérisé en ce qu'**on utilise de 2 à 2000 moles d'eau par mole de disulfonate d'arylhydrazine.

**6.** Procédé selon l'une ou plusieurs des revendications 1 à 5, **caractérisé en ce qu'**on utilise en tant qu'acide inorganique HCl, $H_2SO_4$, $H_3PO_4$, à l'état anhydre ou en solution aqueuse.

**7.** Procédé selon l'une ou plusieurs des revendications 1 à 6, **caractérisé en ce qu'**on utilise de 1 à 30 moles d'acide inorganique par mole de disulfonate d'arylhydrazine.

**8.** Procédé selon l'une ou plusieurs des revendications 1 à 7, **caractérisé en ce qu'**on utilise en tant que solvant organique inerte un hydrocarbure aliphatique ayant de 5 à 25 atomes de carbone, un hydrocarbure aromatique ayant de 6 à 12 atomes de carbone, un alcool aliphatique ayant de 1 à 12 atomes de carbone, un polyalkylèneglycol ayant de 2 à 6 atomes de carbone par fragment alkylène, un dialkyléther ayant de 2 à 20 atomes de carbone par fragment alkyle, un éther dialkylique d'un polyalkylèneglycol ayant de 1 à 6 atomes de carbone par fragment alkylène et de 1 à 4 atomes de carbone par fragment alkyle, un ester alkylique en $C_1$-$C_4$ d'un acide carboxylique aliphatique en $C_1$-$C_6$, un di (alkyle en $C_1$-$C_4$)amide d'un acide carboxylique aliphatique en $C_1$-$C_6$, un nitrile, un dialkylsulfoxyde ayant de 1 à 4 atomes de carbone par fragment alkyle, une dialkylsulfone ayant de 1 à 4 atomes de carbone par fragment alkyle, une imidazolinone, une pyrrolidone ou un mélange de ceux-ci.

**9.** Procédé selon l'une ou plusieurs des revendications 1 à 8, **caractérisé en ce qu'**on utilise en tant que solvant organique inerte le méthanol, l'éthanol, le n-propanol, l'isopropanol, le n-butanol, l'isobutanol, le tert-butanol, le n-pentanol, l'isopentanol, le n-hexanol, l'isohexanol, le n-octanol, l'isooctanol, le n-décanol, l'isodécanol, le n-dodécanol, l'éthylèneglycol, le glycérol, le diéthylèneglycol, le tétraéthylèneglycol, l'éther diméthylique du diéthylèneglycol, l'éther diméthylique du tétraéthylèneglycol, le diméthylformamide, le diéthylformamide, le diméthylacétamide ou un mélange de ceux-ci.

**10.** Procédé selon l'une ou plusieurs revendications 1 à 9, **caractérisé en ce qu'**on utilise en tant que solvant organique inerte le méthanol, l'éthanol, le n-propanol, l'isopropanol, le n-butanol, l'isobutanol, le tert-butanol, le n-pentanol, l'isopentanol, le n-hexanol, l'isohexanol, le n-octanol, l'isooctanol, le n-décanol, l'isodécanol ou un mélange de ceux-ci.

**11.** Procédé selon l'une ou plusieurs des revendications 1 à 10, **caractérisé en ce qu'**on sépare le sel d'arylhydrazine sous forme d'une phase organique qui contient le sel d'arylhydrazine dissous dans le solvant organique.